# EUROPEAN PATENT SPECIFICATION

(11) **EP 0 395 106 B1**
(45) Date of publication and mention of the grant of the patent: **06.07.1994**
(21) Application number: 90108113.3
(22) Date of filing: 27.04.1990
(51) Int. Cl.: C12P 19/00, C07H 17/00, C12N 1/20, A01N 63/00, A61K 31/71, C12P 19/60

(54) **Demethylallosamidin and a process for production thereof**
Demethylallosamidin und ein Verfahren zu seiner Herstellung
Déméthylallosamidine et procédé pour sa production

(30) Priority: 27.04.1989 JP 105796/89; 23.03.1990 JP 71972/90
(43) Date of publication of application: 31.10.1990
(73) Proprietor: AJINOMOTO CO., INC., Tokyo 104 (JP)
(72) Inventor: Yamada, Yasuhiro, Ikeda-Shi, Osaka (JP); Sakuda, Shohei, Minoo-shi, Osaka (JP); Takayama, Seiji, c/o Central Research Lab., Kawasaki-ku, Kawasaki-shi, Kanagawa-Ken (JP)
(74) Representative: Strehl Schübel-Hopf Groening & Partner

(56) References cited:
- CHEMICAL ABSTRACTS, vol. 109, no. 9, 29 August 1988, Columbus, OH (US); S. SAKUDA et al., p. 311, no. 69316u#
- CHEMICAL ABSTRACTS, vol. 108, no. 15, 11 April 1988, Columbus, OH (US); S. SAKUTA, p. 348, no. 127448f#
- JOURNAL OF ANTIBIOTICS, vol. XL, no. 3, March 1987; S. SHOHEI et al., pp. 296-300#
- CHEMICAL ABSTRACTS, vol. 111, no. 9, 28 August 1989, Columbus, OH (US); K. DICKINSON et al., pp. 333-334, no. 73568m#

## Description

The present invention relates to a novel allosamine derivative, an antifungal composition, an insecticidal composition and a chitinase inhibitor comprising the same as an effective ingredient.

Furthermore, the present invention relates to a process of producing the novel allosamidin derivative and to a microorganism Streptomyces sp. AJ 9463 (Ferm BP-2801) capable of producing this compound.

Chitin is the main component of the cell wall of fungi (G.W. Gooday and A. Trinci, Symposia of the Society for General microbiology, 30, 207-251, 1980). It is considered that balanced synthesis and decomposition of chitin takes place during repeated division and proliferation of fungi (G.W. Gooday et al., "Chitin in Nature and Technology", pp. 83-91, edited by R.A.A. Muzzarelli et al., Plenum Press, New York, 1986).

Moreover, chitin is the main component of the cuticule of insects and it is also known that synthesis and decomposition of chitin are subtly controlled during the course of ecdysis and growth of insects (K.J. Kramer et al., "Comprehensive Insect Physiology Biochemistry and Pharmacology", vol. 3. p. 75, edited by G.A. Kerkut and L.I. Gilbert, Pergamon Press, Inc., New York, 1985)

It is known that the biosynthesis and decomposition of chitin are regulated mainly by two enzymes, namely chitin synthase and chitinase.

Consequently, it is expected that inhibitors of these enzymes would be a new type of antifungal agents or insect growth regulators (insecticides).

In fact, polyoxin, an inhibitor of chitin synthase, has been provided for actual use as antifungal agent in agricultural fields. Also inhibitory activity of the ecdysis of insects in vivo is reported. (E. Cohen and J.E. Cashida, Pestic. Biochem. Physiol., 17, 301-306, 1982).

Heretofore only allosamidin has been found as inhibitor for chitinase (S. Sakuda et al., J. Antibiotics, 40, 296-300, 1987), which strongly inhibits endo type chitinase derived from insects and is thus expected to be useful as an insecticidal or acaricidal agent (Japanese Patent Application Laid-Open No. 62-207294).

However, allosamidin shows only weak inhibitory activity for chitinase derived from fungi so that it was not considered to be useful as antifungal agent.

Therefore, the present inventors have made extensive investigations to provide inhibitors for fungi-derived chitinase from naturally occurring sources and as a result, have found that certain allosamidin-producing bacteria produce a compound which exhibits extremely potent inhibitory activity.

The present invention relates to a compound represented by the following formula (I): 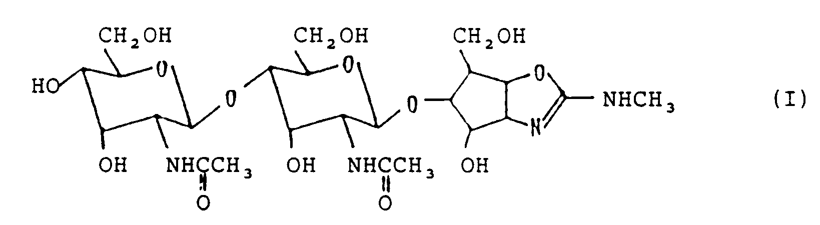
and an antifungal composition, a chitinase inhibitor composition and an insecticidal composition comprising this compound as effective ingredient.

The present invention also provides a process for the production of this compound which comprises culturing bacteria of Streptomyces sp. capable of producing the compound according to claim 1 and isolating said compound from the culture and, further, a microorganism, i. e. Streptomyces sp. AJ 9463 (Ferm BP-2801).

The compound of formula (I) is designated as demethylallosamidin since the compound has the same structure as allosamidin except for the removal of one methyl group in the residue -N(CH₃)₂ of allosamidin. The compound of the formula (I) therefore carries the group -NHCH₃.

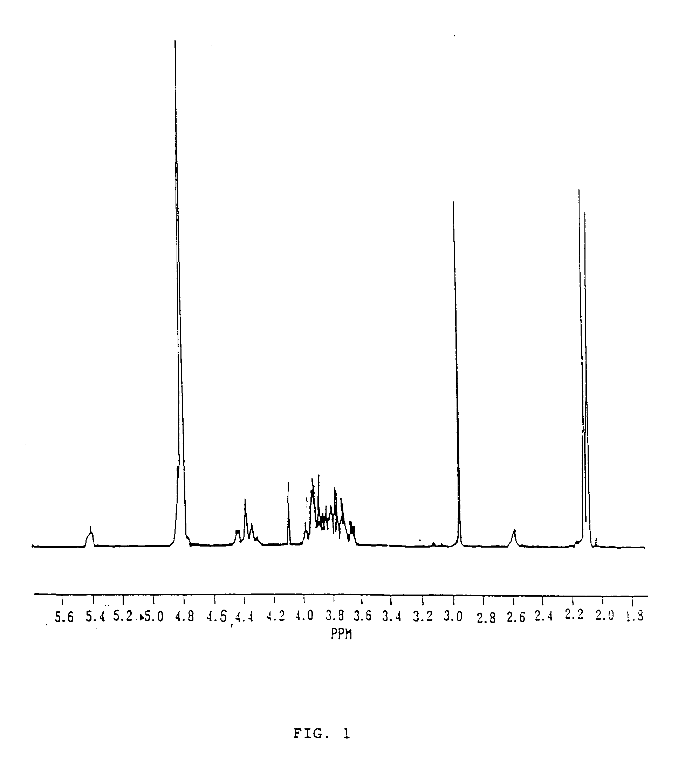

Fig. 1 shows a ¹H-NMR spectrum of demethylallosamine. The bacteria strain capable of producing demethyallosamidin has been identified to belong to Streptomyces sp. based on its bacteriological properties. Thus, it grows aerobically in ISP 3 medium used for the identification of Actinomyces, forms aerial mycelium, forms no sporangium, and forms but does not divide substrate mycelium. Its substrate mycelium forms a long spore chain. The Sporophore is not whirled. In the cell wall LL-Diaminopimelic acid is contained, no characteristic sugar is present and the phospholipid is of type PII. As an example for a strain of Streptomyces sp. capable of producing demethylallosamidin of the present invention, Streptomyces sp. AJ 9463 (FERM P-10642, FERM BP-2801) is given.

To produce demethylallosamidin, bacteria capable of producing the same, for example, Streptomyces sp. FERM P-10642 (FERM BP-2801) is cultured in an appropriate medium and demethylallosamidin is isolated from this medium.

The method for culturing may be carried out in any manner suited for the culture of microorganisms. However, deep culture in a liquid medium is generally preferred. Any medium may be employed for the culture as long as it contains nutrient sources which can be utilized by the producing bacteria. Examples for carbon sources are glucose, fructose, starch, dextrin, etc. Examples for nitrogen sources are meat extract casein, gluten, yeast extract, soybean powders, corn steep liquor, urea, ammonium sulfate, ammonium phosphate, etc. In addition, inorganic salts such as sodium hydrogenphosphate, magnesium sulfate, calcium carbonate, etc. may also be used, if necessary. Furthermore, a small quantity of silicone compounds, higher alcohols, vegetable oils, etc. may be supplemented if foaming is vigorous during culturing.

The temperature for the culture may be advantageously in the range from 20 to 35 °C, most preferably at about 27 °C. The time period for the culture may be advantageously about 1 to 10 days but it may be appropriately varied depending upon the culture conditions.

Demethylallosamidin produced by the culture is accumulated mainly within the cells. Therefore, in general, it will be isolated and purified from the cells by means of centrifugation, filtration, etc., in any manner conventionally used for the isolation of antibiotics. The isolation and purification may be carried out by solvent extraction with a lower alcohol such as methanol, n-butanol, etc.; by adsorption column chromatography using silica gel, diatomaceous earth, avicel, alumina, etc.; by gel filtration using TOYO PEARL HW 40 (carrier for gel filtration manufactured by Toyo Soda Mfg. Co. Ltd.), etc.; by various ion exchange chromatographies; by reverse phase partition column chromatography and by HPLC using octadodecylated (ODS) silica gel as carrier; furthermore, by counter current partition, and by means for purification such as crystallization, recrystallization, etc.; which may be in order or in appropriate combination.

The thus isolated and purified demethylallosamidin exhibits potent inhibitory activity for fungi-derived chitinase which is, per unit weight of demethylallosamidin, 100 times as strong as that of allosamidin.

Furthermore, cytotoxicity of demethylallosamidin is low. Thus, no growth inhibition of mouse ascites mammary cancer cells or human leukemia cells was noted in a dose of 1 mg/ml.

The demethylallosamidin of the present invention is applied as effective ingredient of antifungal compositions, chitinase inhibitor compositions or insecticidal compositions. These compositions may be prepared by conventional means and methods and by using conventional ingredients and additives.

The preparation may have any form, it may be a solution, an emulsion, a suspension, a paste, powders, and the like. Hereafter the present invention is described referring to the example below.

### Example

### (1) Preparation of demethylallosamidin

A medium (pH 7.2)composed of glucose (12 g), meat extract (1.2 g), peptone (2.4 g), yeast extract (1.2 g) and water (1.2 liter) was prepared and 100 ml each of the medium was separately charged in 12 Erlenmeyer flasks having each a volume of 500 ml.

After sterilization at 120 °C for 20 minutes, one platinum loop of slant cultured cells of Streptomyces sp. AJ 9463 (FERM P-10642 (FERM BP-2801)) was inoculated on each medium. Shake culture was carried out at 28 °C for 3 days to give seed mother liquor. Furthermore, 60 liters of a medium composed as described above was prepared and charged in a culture tank of 100 liter volume followed by sterilization at 120 °C for 30 minutes. 1.2 liters of the seed mother liquor described above were added to the medium followed by culturing at 27 °C for 5 days. During the culture, the agitation rate was 200 rpm and the air velocity was 60 liters/min. After celite had been added to the thus obtained culture, the culture was filtered through a funnel to give a mixture of the cells and celite. Methanol (8 liters) was added to the mixture. After thoroughly stirring, the mixture was settled overnight. The mixture was filtered to give a methanol extract. After the extract had been concentrated to about 1 liter under reduced pressure, distilled water was added to the residue in an amount to yield a whole liquid volume of 6 liters. The whole amount was adsorbed onto an activated charcoal column (600 ml volume). After washing the column with distilled water (1.8 liters), elution was performed sequentially with 10 % ethanol (3 liters), 25 % ethanol (3 liters) and 50 % ethanol (3 liters). The activity was mainly observed in the fraction eluted with 25 % ethanol. After the fraction had been concentrated to about 2 liters under reduced pressure, acetic acid was added to the concentrate in order to adjust the pH of the liquid to 3.8. The liquid was adsorbed onto a SP-Sephadex C-25 cation exchange column (160 ml volume) equilibrated with 50 mM ammonium acetate/acetic acid (pH 5.0). A one step elution was performed with the same buffer and fractions were taken every 8 ml. With respect to each fraction, the activity was determined and fractionated into two fractions: Fraction Nos. 55 to 64 having a weak specific activity and Fraction Nos. 65 to 75 having a strong specific activity. Among them, the component of Nos. 55 to 64 was identified to be allosamidin, based on its various physicochemical properties determined by FABMS, NMR, etc. The fractions Nos. 65 to 75 were desalted with a small quantity of activated charcoal, then concentrated under reduced pressure and finally purified by high performance liquid chromatography (moving phase: 10 mM ammonium acetate/ammonia (pH 8.9), flow rate: 1.0 ml/min) using a weak cationic exchange column (Asahipak ES-502C). Detection was carried out by UV absorption at 220 nm and two peaks showing a retention time (t_{R}) of 7.0 minutes and 8.8 minutes were observed and the respective fractions were freeze dried. Among them, the substance having t_{R} of 8.8 minutes was identified to be allosamidin based on its various physicochemical properties. The other substance showing t_{R} of 7.0 minutes was identified to be demethylallosamidin, by giving a single peak on HPLC when using various ion exchange columns and based on its various physicochemical properties described below.
(1) Appearance: white powder
(2) Molecular formula: C₂₄H₄₀N₄O₁₄
(3) FABMS: m/z 609 (M+H)⁺, glycerol matrix
(4) UV absorption spectrum: terminal absorption (in 0.1 N acetic acid)
(5) ¹H-NMR spectrum: as shown in Fig. 1 (600 MHz, D₂O + AcOD)

### (2) Determination of chitinase inhibitory activity of demethylallosamidin

Demethylallosamidin of formula (I) prepared as described above exhibits a potent inhibitory activity for fungi-derived chitinase.

### (i) Preparation of a chitinase solution

In a Sakaguchi flask of 2 liters 1 liter of 25mM MES (Nakarai Chemical Co., Ltd.) buffer containing 0.1 % β-mercaptoethanol and 200 g of baker's yeast (Kanegafuchi Chemical Industry Co., Ltd.) was charged followed by shaking at 30 °C at 120 spm for 2 hours. The cells were removed by centrifugation (0 °C, 10 mins. 12,000 g). The resulting supernatant was concentrated to 200 ml by ultrafiltration (TOYO ULTRAFILTER UP-20). After concentration, 400 ml of citrate buffer (0.15 M citric acid + 0.15 M sodium citrate, pH 3.0) were added to the concentrate. After the formed precipitates had been removed by centrifugation (0 °C, 10 mins., 12,000 g), the resulting supernatant was again concentrated to 40 ml by ultrafiltration. The concentrate was stored at 4 °C and used as chitinase solution for the assay.

### (ii) Preparation of the enzyme substrate

After 10 ml of 10 % acetic acid had been slowly added to 0.5 g of chitosan (PFANSTIEHL LABORATORIES INC.), the mixture was kneaded in a mortar in order to become gel-like. After allowing to stand at room temperature overnight, 45 ml of methanol were added while thoroughly stirring the mixture. Thereafter, the mixture was filtered through a gauze and 0.75 ml of ³H-labelled acetic anhydride (NET 018A10 ACETIC ANHYDRIDE 5 mCi) was added to the filtrate obtained. The formed agar-like chitin was homogenized with a homogenizer, collected on a glass filter (Whatmann GF/B) and suspended in 10 ml of citrate buffer (pH 3.0). This chitin suspension (2.3 »Ci/ml, chitin suspension) was used as substrate for the assay.

### (iii) Method for determination of chitinase inhibitory activity

90 »l of the chitinase solution and 10 »l of the ³H-chitin suspension were charged in an Eppendorf tube and reacted at 37 °C for 3 hours. As blank 90 »l of citrate buffer (pH 3.0) were used in place of 90 »l of the enzyme solution in this case and reacted in a similar manner. After the reaction 100 »l of 10 % trichloroacetic acid were added and the reaction solution was passed through a glass filter (Whatman GF/B). After 10 ml of scintillant solution had been added to the thus obtained filtrate, its radioactivity (dpm) was determined. The difference between the thus determined value and the radioactivity of the blank was defined as chitinase activity.

The scintillant solution was prepared by dissolving 4 g of Ominiflour (Daiichi Chemical) in 500 ml of toluene and adding 500 ml of Triton X-100 (Nakarai Chemical Co., Ltd.) to the solution.

Allosamidin and demethylallosamidin each were dissolved in 0.1 N acetic acid solutions and 10 »l each of the solutions having the respective concentrations were added to the reaction system described above. Additionally, 10 »l of 0.1 N acetic acid were added to the blank and to a system to which no inhibitor was added, followed by the reaction described above.

The inhibitory activity was calculated according to the following equation 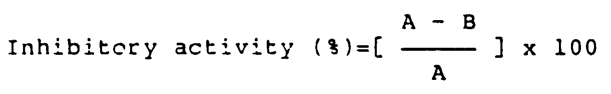
A: chitinase activity (dpm) when no inhibitor was added.
B: chitinase activity (dmp) when the inhibitor was added.

In any of the measurements, 3 series of runs were conducted and the mean value was determiend.

### (iv) Results

The results are shown in Table 1.

**Table 1**

| | Inhibition Rate (%) | |
|---|---|---|
| Demethylallosamidin (»g) | 0.0 | - |
| | 0.8 | 87.1 |
| | 0.4 | 84.6 |
| | 0.2 | 75.2 |
| | 0.1 | 73.9 |
| | 0.05 | 61.1 |
| Allosamidin (»g) | | |
| | 4 | 58.5 |
| | 2 | 47.6 |
| | 1 | 36.0 |

### (3) Influence of demethylallosamidin on the growth of mold

Demethylallosamidin was added to a culture medium of mold in order to examine its influence. Firstly, Fusarium nivale (ATCC 42308) was precultured in PDA medium (manufactured by Nissui Pharmaceutical Co., Ltd.) to form spores. The thus obtained spores were suspended in physiological saline. The suspension was filtered through cotton in order to obtain a spore suspension free of hyphae.

A fixed amount of the resulting suspension was inoculated on media supplemented with demethylallosamidin (0.80 »g/ml and 0.16 »g/ml, respectively) and on demethylallosamidin-free medium (control), respectively. Change in subsequent incubation was observed in the passage of time. The observaton was performed as follows: after definite periods of time samples were taken, from which microscopic photographs were made and the length and branching degree of the hyphae was determined. The branching degree of the hyphae is the mean value of the branch intervals of the hyphae. In any case, the incubation was carried out at 28 °C at 120 strokes/min. by charging 1 ml each of the medium (0.03 % of yeast extract, 0.03 % of malt extract (both manufactured by Difco Co., Ltd.), 0.05 % of polypeptone (manufactured by Daigo Nutrient Co., Ltd.) and 0.1 % of glucose) in a testing tube with a cotton stopper (diameter of 11 mm).

The length and branching degree of the hyphae after 32 hours are shown in Table 2. By the addition of demethylallosamidin, the length of hyphae obviously increased and the branching degree decreased. Particularly at the top of the hyphae the decrease in the branching degree was remarkable.

**Table 2**

| Concentration of demethylallosamidin of (»g/ml) | Length Hypha (»m) | Branching Degree of Hypha (»m) |
|---|---|---|
| 0 | 143 | 49.2 |
| 0.16 | 364 | 72.8 |
| 0.80 | 576 | 74.2 |

### (4) Cytotoxicity of demethylallosamidin

i) Mouse ascitic mammary cancer cells FM3A were inoculated in an amount of 1 x 10⁵/ml on Dulbecco modified MEM medium supplemented with 10 % fetal calf serum and demethylallosamidin was added thereto in a concentration of 1 mg/ml followed by stationary culture at 37 °C for 4 days. Microscopic observation of the cultured mouse ascitic mammary cancer cells FM3A reveals that no growth inhibition was noted at all.
ii) Human leukemia cells K562 were inoculated in an amount of 1 x 10⁵/ml on FRMI 1640 medium supplemented with 10 % fetal calf serum and demethylallosamidin was added thereto in a concentration of 1 mg/ml followed by stationary culture at 37 °C for 4 days. Microscopic observation of the cultured human leukemia cells K562 reveals that no growth inhibitaion was noted at all.

The compound of the present invention has an extremely potent inhibitory activity against fungi-derived chitinase. Accordingly, antifungal agent and chitinase inhibitor having excellent pharmaceutical effects can be provided. In addition, this compound can be produced by fermentation and can thus be produced in large quantities.

## Claims

1. Compound represented by the following formula:

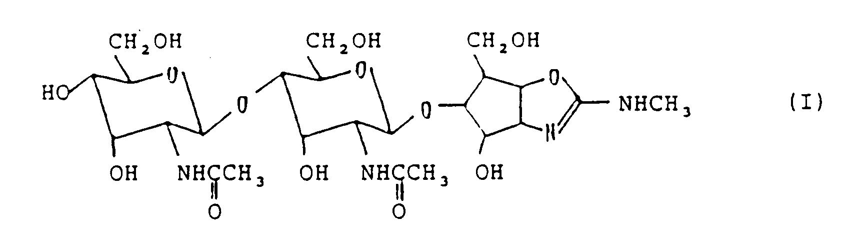

2. A process for the production of the compound according to claim 1, which comprises culturing bacteria of Streptomyces sp. capable of producing the compound according to claim 1 and isolating said compound from the culture.

3. A process according to claim 2, wherein the Streptomyces sp. is Streptomyces sp. AJ 9463 (Ferm P-10642, Ferm BP-2801).

4. Streptomyces sp. AJ 9463 (Ferm P-10642, Ferm BP-2801).

5. An antifungal composition or chitinase inhibitor composition comprising the compound according to claim 1 as effective ingredient.

6. An insecticidal composition, comprising the compound according to claim 1.

## Patentansprüche

1. Verbindung, die durch die folgende Formel dargestellt ist:

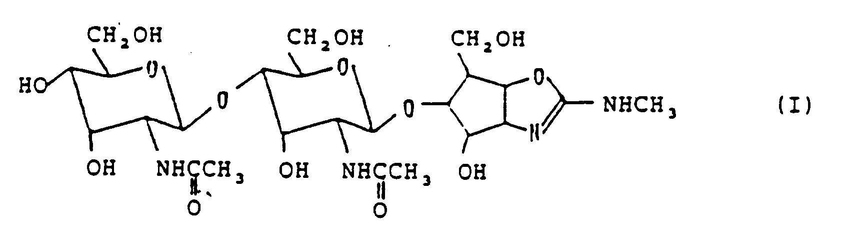

2. Verfahren zur Herstellung der Verbindung nach Anspruch 1, das darin besteht, daß zur Bildung der Verbindung gemäß Anspruch 1 befähigte Bakterien einer Streptomyces-Species gezüchtet werden und die Verbindung aus der Kultur isoliert wird.

3. Verfahren nach Anspruch 2, wobei die Streptomyces-Species Streptomyces-AJ 9463 (Ferm P-10642, Ferm BP-2801) ist.

4. Streptomyces sp. AJ 9463 (Ferm P-10642, Ferm BP-2801).

5. Fungizide Zusammensetzung oder Chitinase-Inhibitor-Zusammensetzung, enthaltend die Verbindung nach Anspruch 1 als Wirksubstanz.

6. Insektizide Zusammensetzung, enthaltend die Verbindung gemäß Anspruch 1.

## Revendications

1. Composé représenté par la formule suivante :

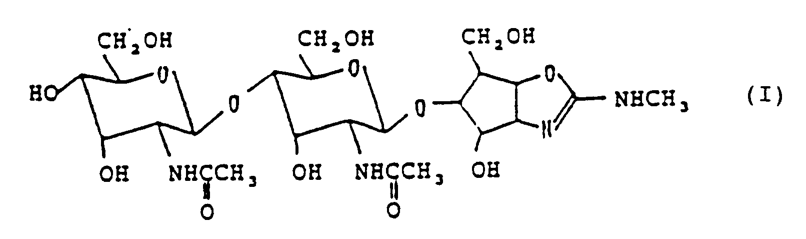

2. Procédé pour la préparation du composé selon la revendication 1, lequel comprend la culture de bactéries de Streptomyces sp. capables de produire le composé selon la revendication 1 et l'isolement dudit composé de la culture.

3. Procédé selon la revendication 2, dans lequel le Streptomyces sp. est le Streptomyces sp. AJ 9463 (Ferm P-10642, Ferm BP-2801).

4. Streptomyces sp. AJ 9463 (Ferm P-10642, Ferm BP-2801).

5. Composition antifongique ou composition d'inhibiteur de chitinase comprenant le composé selon la revendication 1 en tant que principe actif.

6. Composition insecticide, comprenant le composé selon la revendication 1.
